# EUROPÄISCHE PATENTSCHRIFT

(11) **EP 4 138 738 B1**
(45) Veröffentlichungstag und Bekanntmachung des Hinweises auf die Patenterteilung: **19.11.2025**
(21) Anmeldenummer: 22702174.8
(22) Anmeldetag: 17.01.2022
(51) Int. Cl.: A61F 2/30

(54) **COMPUTERIMPLEMENTIERTES VERFAHREN ZUM ENTWERFEN EINER KIEFERGELENKSPROTHESE, ENTSPRECHENDES HERSTELLUNGSVERFAHREN, COMPUTERSYSTEM, COMPUTERPROGRAMMPRODUKT, DATENSPEICHERMEDIUM UND ENTSPRECHENDER DATENSTREAM UND WEBSERVER**
COMPUTER-IMPLEMENTED METHOD FOR DESIGNING A TEMPOROMANDIBULAR JOINT PROSTHESIS, CORRESPONDING MANUFACTURING METHOD, COMPUTER SYSTEM, COMPUTER PROGRAM PRODUCT, DATA STORAGE MEDIUM, DATA STREAM, AND WEB SERVER
MÉTHODE DE CONCEPTION D'UNE PROTHÈSE DE L'ARTICULATION TEMPORO-MANDIBULAIRE MISE EN OEUVRE PAR ORDINATEUR, PROCÉDÉ DE FABRICATION, SYSTÈME INFORMATIQUE, PRODUIT LOGICIEL, SUPPORT DE STOCKAGE DE DONNÉES, FLUX DE DONNÉES ET SERVEUR WEB CORRESPONDANT

(30) Priorität: 11.02.2021 DE 102021201278
(43) Veröffentlichungstag der Anmeldung: 01.03.2023
(73) Patentinhaber: Karl Leibinger Asset Management GmbH & Co. KG, 78570 Mühlheim an der Donau (DE)
(72) Erfinder: AKSU, Adem, 78054 VS-Schwenningen (DE); LEIBINGER, Ralf, 78570 Mühlheim/Donau (DE); REINAUER, Frank, 78576 Emmingen-Liptingen (DE); WOLFRAM, Tobias, 63303 Dreieich (DE)
(74) Vertreter: Isarpatent
(86) Internationale Anmeldenummer: PCT/EP2022/050847
(87) Internationale Veröffentlichungsnummer: WO 2022/171381

(56) Entgegenhaltungen:
- CN-A- 110 251 275
- CN-U- 210 749 669
- US-A1- 2014 005 685
- US-A1- 2020 297 495
- US-B2- 8 166 627

## Beschreibung

Die vorliegende Erfindung betrifft ein computerimplementiertes Verfahren zum Entwerfen einer implantierbaren Kiefergelenksprothese mit einem ersten Implantatteil (Unterkieferkomponente) mit einem künstlichen Gelenkkopf, welches an einem Unterkiefer befestigbar ist, und einem zweiten Implantatteil (Craniumkomponente) mit einer Gelenkfläche, welches an einem Cranium befestigbar ist, wobei die Gelenkfläche ein Widerlager für den künstlichen Gelenkkopf bildet. Außerdem betrifft die Erfindung ein entsprechendes Verfahren zur Herstellung einer implantierbaren Kiefergelenksprothese. Zudem betrifft die vorliegende Erfindung ein entsprechendes Computersystem, ein entsprechendes Computerprogrammprodukt, ein entsprechendes computerlesbares, nicht-flüchtiges Datenspreichermedium, einen entsprechender Datenstream und einen entsprechenden Webserver.

Die US 2020/297495 A1 offenbart ein computerimplementiertes Verfahren zum Entwerfen einer Gelenksprothese mit den Schritten: Bereitstellen einer 3-dimensionalen, 3D-, Darstellung des Patienten; Bereitstellen eines 3-dimensionalen, 3D-, Modells für zumindest eine funktionale Gruppe der Prothese; Anordnen des bereitgestellten 3D-Modells bezüglich der 3D-Darstellung an einer zu behandelnden Seite der 3D-Darstellung; Anpassen des bereitgestellten 3D-Modells in Größe und Ausrichtung an die anatomischen Verhältnisse der 3D-Darstellung an der zu behandelnden Seite; Extrahieren einer Fossa-Gleitfläche aus dem angepassten 3D-Modell; Anordnen der extrahierten Fossa-Gleitfläche an der zu behandelnden Seite; und Ausgeben von Konstruktionsdaten für die Gelenksprothese.

Das Kiefergelenk, welches im medizinischen Sprachgebrauch auch als "Temporomandibuläres Gelenk", oder, nach dessen englischer Bezeichnung, als TMJ ("temporomandibular joint") bezeichnet wird, ist eines der meist genutzten und wichtigsten Gelenke im menschlichen Körper. Es spielt eine wesentliche Rolle in der Bewegungsführung der Mandibula (des Unterkiefers), beim Kauen, Sprechen, Schlucken und der Stressbewältigung. Dabei ist es sogar im Schlaf durch die Schluckbewegung ständig in Bewegung.

Aufgrund seiner vielfältigen Anatomie und seiner sechs Freiheitsgrade gehört das Kiefergelenk auch zu den komplexesten Gelenken des menschlichen Körpers. Damit das Kiefergelenk seine Bewegungsabläufe durchführen kann, müssen die Form der Gelenkflächen, der Zustand des Gebisses, die Zahnstellung, die Zahnform und die Kaumuskulatur ein funktionales System bilden, welches durch die Vielzahl an Komponenten einer gewissen Störungsanfälligkeit unterliegt. Das linke und rechte Kiefergelenk arbeiten dabei stets zusammen und sind in der Regel spiegelsymmetrisch zueinander geformt.

Treten im Bereich des Kiefergelenks jedoch Störungen, wie z.B. Funktionseinschränkungen oder Erkrankungen, auf, kann es zu erheblichen Beeinträchtigungen der Lebensqualität im Alltag von Patienten kommen. Eine Wiederherstellung von korrekten Gelenkstrukturen ist hierbei teilweise nur im Wege einer chirurgischen Lösung, also durch Resektion (d.h. Entfernung) des gestörten Gelenks und dessen Ersatz durch eine Kiefergelenksprothese, möglich.

Eine Kiefergelenksprothese ist beispielsweise aus der EP 3 003 225 B1 bekannt, welche einen Mechanismus bereitstellt, der eine kombinierte Translations- und Rotationsbewegung zwischen Schädel und Unterkiefer gestattet, bei der eine gleitende Bewegung zwischen Oberflächen stattfindet.

Das Entwerfen von Kiefergelenksprothesen ist jedoch eine anspruchsvolle Aufgabe, da jede Prothese stets nur eine unvollkommene Näherung an die nativen Strukturen darstellt und die Prothese üblicherweise am Reißbrett bzw. am Computer entworfen wird, ohne dass dabei die funktionale Interaktion der artikulierenden Komponenten geprüft werden können.

Es ist die Aufgabe der vorliegenden Erfindung, ein verbessertes Verfahren zum Entwerfen einer Kiefergelenksprothese, ein verbessertes Herstellungsverfahren für eine Kiefergelenksprothese, sowie ein Computersystem zum Entwerfen einer Kiefergelenksprothese bereitzustellen.

Die Aufgabe wird durch ein computerimplementiertes Verfahren mit den Merkmalen des Anspruchs 1 gelöst. Somit wird ein Verfahren zum Entwerfen einer Kiefergelenksprothese für eine zu behandelnde Schädelseite eines Patienten bereitgestellt, mit den Schritten:
Bereitstellen einer 3-dimensionalen, 3D-, Darstellung des Schädels umfassend zumindest die Kiefergelenke des Patienten;
Bereitstellen eines 3-dimensionalen, 3D-, Modells für zumindest eine funktionale Gruppe der Kiefergelenksprothese, wobei die funktionale Gruppe zumindest einen Kondylenkopf und eine Fossa-Gleitfläche für den Kondylenkopf umfasst;
Anordnen des bereitgestellten 3D-Modells bezüglich der 3D-Darstellung des Schädels an einer Referenz-Schädelseite der 3D-Darstellung, wobei die Referenz-Schädelseite eine der zu behandelnden Schädelseite gegenüberliegende Schädelseite ist;
Anpassen des bereitgestellten 3D-Modells in Größe und Ausrichtung an die anatomischen Verhältnisse der 3D-Darstellung an der Referenz-Schädelseite;
Spiegeln eines Kondylenkopf-Modells von der Referenz-Schädelseite an die zu behandelnde Schädelseite, wobei das Kondylenkopf-Modell entweder durch einen Kondylenkopf der 3D-Darstellung auf der Referenz-Schädelseite oder durch mindestens einen Teil des Kondylenkopfs des 3D-Modells gebildet wird;
Extrahieren einer Fossa-Gleitfläche aus dem angepassten 3D-Modell;
Anordnen der extrahierten Fossa-Gleitfläche an der zu behandelnden Schädelseite;
Anpassen des gespiegelten Kondylenkopf-Modells sowie der Fossa-Gleitfläche aneinander und/oder an eine Anatomie der 3D-Darstellung auf der zu behandelnden Schädelseite;
Ausgeben von Konstruktionsdaten für die Kiefergelenksprothese (insbesondere in Form eines Kiefergelenksprothese-3D-Modells), welche zumindest das angepasste gespiegelte Kondylenkopf-Modell sowie die angepasste Fossa-Gleitfläche umfassen.

Insbesondere die grundlegende Idee, Strukturen an der gesunden Referenz-Schädelseite zu verwenden, um ein geeignetes 3D-Modell anzupassen und daraufhin eine Spiegelung auf die zu behandelnde Schädelseite durchzuführen, ermöglicht es, möglichst viele Informationen von der gesunden Referenz-Schädelseite für das Entwerfen der Prothese zu berücksichtigen. So kann nicht nur eine gute Zusammenwirkung der miteinander artikulierenden Implantatteile der Kiefergelenksprothese an der zu behandelnden Schädelseite für sich genommen erzielt werden, sondern darüber hinaus auch eine möglichst gleichlaufende und gleichartige Bewegung auf beiden Schädelseiten.

Unter dem Entwerfen einer Kiefergelenksprothese soll insbesondere verstanden werden, die Kiefergelenksprothese derart zu planen oder zu designen oder auszulegen, dass Konstruktionsdaten vorliegen, die am Ende lediglich noch durch entsprechende Maschinen oder rein mechanische Handgriffe in die tatsächliche Kiefergelenksprothese umgesetzt zu werden brauchen.

Wann immer hierin von einem "Patienten" gesprochen wird, versteht es sich, dass damit sowohl weibliche als auch männliche Patienten gemeint sind.

Unter der funktionalen Gruppe der Kiefergelenksprothese soll die Gruppe von Elementen oder Abschnitten verstanden werden, die bei der Verwendung der Kiefergelenksprothese anstelle eines natürlichen Kiefergelenks miteinander artikulieren, d.h. ineinander eingreifen, aneinander reiben, sich gegenseitig abstützen, Gegenkräfte aufeinander ausüben und dergleichen. Mit anderen Worten soll die funktionale Gruppe zumindest diejenigen Elemente enthalten, welche dafür ausgelegt sind, eine Bewegung des Kiefergelenks durch Interaktion miteinander zu ermöglichen. Bei der Kiefergelenksprothese ist dies somit zumindest der Kondylenkopf (bzw. ein diesem entsprechendes Element) sowie eine Fossa-Gleitfläche der zugehörigen Fossa articularis (bzw. eines dieser entsprechenden Elements).

Die Bezeichnung "Schädel", wie sie hierin verwendet wird, soll insbesondere das Cranium, die Maxilla sowie den Unterkiefer (mandibula) umfassen.

Die Begriffe "Fossa" und "Fossa articularis" werden hierin gleichbedeutend verwendet. Ebenso werden die Begriffe "Kiefergelenksköpfchen", "Kondylus" und "Kondylenkopf" gleichbedeutend verwendet.

Bevorzugte Weiterbildungen der erfindungsgemäßen Gegenstände ergeben sich aus den Unteransprüchen sowie aus der Beschreibung, insbesondere mit Bezug auf die Zeichnungen.

Gemäß einigen bevorzugten Ausführungsformen, Varianten oder Weiterbildungen von Ausführungsformen umfasst das Verfahren ein Entwerfen eines Fossa-Grundkörpers der Kiefergelenksprothese derart, dass der Fossa-Grundkörper die Fossa-Gleitfläche trägt. Hierbei kann insbesondere vorgesehen sein, dass die Fossa-Gleitfläche der späteren Kiefergelenksprothese aus einem Kunststoff, insbesondere einem Polyethylen, ausgebildet wird, mit welchem der Fossa-Grundkörper, der vorzugsweise aus Titan ausgebildet ist, als Material-Verbund ausgeführt wird. Beispielsweise kann die Fossa-Gleitfläche mittels eines Sinterprozesses mit dem Fossa-Grundkörper verbunden werden.

Wann immer hierin von einer Ausbildung von Teilimplantaten, Abschnitten oder Elementen aus Titan die Rede ist, versteht es sich, dass statt Titan auch eine geeignete Titanlegierung verwendet werden kann.

Gemäß einigen bevorzugten Ausführungsformen, Varianten oder Weiterbildungen von Ausführungsformen wird das angepasste gespiegelte Kondylenkopf-Modell als Teil einer separaten Craniumkomponente der Kiefergelenksprothese entworfen (oder: designt, oder: ausgelegt) und die angepasste Fossa-Gleitfläche der Kiefergelenksprothese als Teil einer separaten Unterkieferkomponente der Kiefergelenksprothese entworfen (oder: designt, oder: ausgelegt). Die Craniumkomponente und die Unterkieferkomponente können auch als separate Teilimplantate (oder: Implantat-Teile) bezeichnet werden. Die Ausbildung von Craniumkomponente und Unterkieferkomponente als voneinander separate Teilimplantate ermöglicht zunächst eine große Bewegungsfreiheit der beiden Teilimplantate zueinander, welche später durch geeignete Mittel oder Maßnahmen, z.B. einen Faden, durch einen Arzt zielführend eingeschränkt werden kann. Zudem wird auf diese Weise die Herstellung vereinfacht, da die beiden Teilimplantate separat voneinander hergestellt werden können.

Gemäß einigen bevorzugten Ausführungsformen, Varianten oder Weiterbildungen von Ausführungsformen werden die Craniumkomponente und die Unterkieferkomponente jeweils derart entworfen, dass sie eine jeweilige Öse (Craniumkomponentenöse und Unterkieferkomponentenöse) umfassen, mittels welcher beider die Craniumkomponente und die Unterkieferkomponente durch einen Faden oder ein Band miteinander koppelbar sind. Vorteilhaft kann ein solcher Faden resorbierbar ausgebildet sein und an der in den menschlichen Körper eingesetzten Kiefergelenksprothese befestigt sein, um die Craniumkomponente und die Unterkieferkomponente beweglich miteinander zu koppeln. Dies kann dem Patienten in der anfänglichen Gewöhnungsphase an die Kiefergelenksprothese bei dem Erlernen der richtigen Bewegungen/Artikulationen und Positionen der Kiefergelenksprothese helfen.

Die Erfindung schafft ebenfalls ein Verfahren zur Herstellung einer Kiefergelenksprothese nach Anspruch 5.

Gemäß einigen bevorzugten Ausführungsformen, Varianten oder Weiterbildungen von Ausführungsformen werden zumindest Teile der Kiefergelenksprothese mittels eines generativen (oder: additiven) Verfahrens unter Verwendung von Laserschmelzen erzeugt, insbesondere unter Verwendung von Titan-Laserschmelzen, sodass die entsprechenden Teile vollständig oder zumindest teilweise aus Titan ausgebildet sind. Titan ist ein für Kiefergelenksprothesen gut geeignetes Material, und durch Laserschmelzen lassen sich hochwertige, stabile, und gleichzeitig dem zugrundeliegenden 3D-Modell formgetreue Implantate herstellen.

Bevorzugt werden alle aus Titan gefertigten Teile oder Elemente, welche beim bestimmungsgemäßen Gebrauch der Kiefergelenksprothese artikulieren, poliert. Somit verringert sich deren Reibungskoeffizient nochmals beträchtlich, was noch eine bessere Artikulation der Kiefergelenksprothese ermöglicht. Besonders bevorzugt ist es, dass der Kondylenkopf der Kiefergelenksprothese (d.h. dasjenige Element, welches bei der Kiefergelenksprothese diejenigen Funktionen erfüllt, welche bei einem intakten Kiefergelenk der Kondylenkopf erfüllt) poliert wird.

Gemäß einigen bevorzugten Ausführungsformen, Varianten oder Weiterbildungen von Ausführungsformen wird die Fossa-Gleitfläche der Kiefergelenksprothese als Gleitschicht aus einem Kunststoff, insbesondere aus einem Polyethylen, ausgebildet. Bei dem Polyethylen kann es sich insbesondere um UHMWPE handeln. UHMWPE ist ein thermoplastischer Polyethylen mit ultra-hohem Molekülgewicht, beispielsweise von 2 bis 8 Millionen g/mol. Beispiele für UHMWPE werden beispielsweise unter der Markenbezeichnung "Celanese GUR^{®} 1020" vertrieben. Bevorzugt wird die Fossa-Gleitfläche mit Hilfe eines Sinterprozesses mit einem aus Titan gefertigten Fossa-Grundkörper verbunden.

Die Kombination aus miteinander artikulierendem Kondylenkopf aus poliertem Titan und Fossa-Gleitfläche aus UHMWPE hat sich als besonders reibungsarm und daher besonders vorteilhaft herausgestellt.

Gemäß einigen bevorzugten Ausführungsformen, Varianten oder Weiterbildungen von Ausführungsformen weist die Unterkieferkomponente außer einem Kondylenkopf gemäß dem Kondylenkopf-Modell zumindest eine Unterkieferfixationsplatte zum Fixieren der Unterkieferkomponente am Unterkiefer des Patienten auf. Diese ist üblicherweise mit Löchern versehen, damit sie mittels Schrauben an dem Unterkiefer des Patienten befestigt werden kann. Es können aber auch andere Befestigungsmittel vorgesehen sein und die Unterkieferfixationsplatte entsprechend ausgebildet sein. Die Unterkieferkomponente wird bevorzugt einstückig aus Titan gefertigt, besonders bevorzugt durch Titan-Laserstrahlschmelzen.

Gemäß einigen bevorzugten Ausführungsformen, Varianten oder Weiterbildungen von Ausführungsformen weist die Craniumkomponente eine Craniumfixationsplatte zum Fixieren der Craniumkomponente am Cranium des Patienten auf. Diese ist üblicherweise mit Löchern versehen, damit sie mittels Schrauben an dem Cranium des Patienten befestigt werden kann. Es können aber auch andere Befestigungsmittel vorgesehen sein und die Unterkieferfixationsplatte entsprechend ausgebildet sein. Die Unterkieferkomponente wird bevorzugt einstückig aus Titan gefertigt, besonders bevorzugt durch Titan-Laserstrahlschmelzen.

Bevorzugt wird die Craniumkomponente aus einem Verbund von Titan und einem Polyethylen, insbesondere UHMWPE, ausgebildet.

Gemäß einigen bevorzugten Ausführungsformen, Varianten oder Weiterbildungen von Ausführungsformen umfasst das Verfahren neben dem Entwerfen und Herstellen der Kiefergelenksprothese auch einen Schritt des Einsetzens bzw. Implantierens der Kiefergelenksprothese in einen Patienten. Optional kann hierbei vor, während, oder nach dem Einsetzen auch ein (vorzugsweise resorbierbarer) Faden verwendet werden, um die Craniumkomponente und die Unterkieferkomponente durch eine jeweilige Craniumkomponentenöse und Unterkieferkomponentenöse miteinander beweglich zu koppeln.

Die Erfindung stellt außerdem ein Computersystem nach Anspruch 10 bereit, welches zum Durchführen des Verfahrens zum Erstellen der Kiefergelenksprothese ausgelegt ist.

Die Erfindung stellt weiterhin ein Computerprogrammprodukt nach Anspruch 11 bereit, welches ausführbaren Programmcode umfasst, welcher dazu ausgelegt ist, wenn er ausgeführt wird, ein Verfahren gemäß einer Ausführungsform des Verfahrens zum Entwerfen der Kiefergelenksprothese durchzuführen.

Die Erfindung stellt außerdem auch ein computerlesbares, nicht-flüchtiges Datenspeichermedium nach Anspruch 12 bereit, welches ausführbaren Programmcode umfasst, welcher dazu ausgelegt ist, wenn er ausgeführt wird, ein Verfahren gemäß einer Ausführungsform des Verfahrens zum Entwerfen der Kiefergelenksprothese durchzuführen.

Die Erfindung stellt außerdem auch einen Datenstream nach Anspruch 13 bereit, welcher ausführbaren Programmcode umfasst oder dazu ausgelegt ist, einen solchen ausführbaren Programmcode bereitzustellen, wobei der ausführbare Programmcode dazu ausgelegt ist, wenn er ausgeführt wird, ein Verfahren gemäß einer Ausführungsform des Verfahrens zum Entwerfen der Kiefergelenksprothese durchzuführen.

Die Erfindung stellt außerdem einen Webserver nach Anspruch 14 bereit, welcher dazu ausgelegt ist, ein Verfahren gemäß einer Ausführungsform des Verfahrens zum Entwerfen der Kiefergelenksprothese als Web-Service bereitzustellen bzw. zugänglich zu machen.

Die Erfindung wird nachstehend anhand von Ausführungsbeispielen in den Figuren der Zeichnung näher erläutert. In teilweise schematisierter Darstellung zeigen hierbei:
- Fig. 1: ein schematisches Flussdiagramm zum Erläutern eines Verfahrens gemäß einer Ausführungsform der vorliegenden Erfindung;
- Fig. 2: eine 3-dimensionale graphische Darstellung des Schädels eines Patienten;
- Fig. 3a: eine graphische Darstellung eines Unterkiefers sowie eines 3D-Modells für eine funktionale Gruppe einer Kiefergelenksprothese in einer gemeinsamen graphischen Umgebung von vorne;
- Fig. 3b: eine graphische Darstellung des Unterkiefers sowie eines 3D-Modells für eine funktionale Gruppe einer Kiefergelenksprothese in einer gemeinsamen graphischen Umgebung von vorne, nachdem das 3D-Modell an die graphische Darstellung des Unterkiefers angepasst wurde;
- Fig. 4a: eine Abbildung der Situation aus Fig. 3a von der Seite;
- Fig. 4b: eine Abbildung der Situation auf Fig. 3b von der Seite;
- Fig. 5a: eine Ansicht von oben auf eine graphische Darstellung eines Unterkiefers mit einem auf einer gesunden Schädelseite markierten Kondylenkopf und einem defekten Kondylenkopf an einer zu behandelnden Schädelseite;
- Fig. 5b: die Ansicht aus Fig. 5a, wobei ein Kondylenkopf-Modell dem defekten Kondylenkopf überlagert wurde;
- Fig. 6a: eine schematische Detailansicht des Kondylenkopfs des 3D-Modells der funktionalen Gruppe der Kiefergelenksprothese;
- Fig. 6b: eine schematische Detailansicht eines Kondylenkopfs gemäß einem Kondylenkopf-Modell;
- Fig. 7a: eine Detailansicht auf eine Fossa-Gleitfläche des 3D-Modells der funktionalen Gruppe der Kiefergelenksprothese;
- Fig. 7b: die Fossa-Gleitfläche aus Fig. 7a nach ihrer Extraktion aus dem 3D-Modell;
- Fig. 8a: eine graphische Darstellung des Schädels des Patienten nach einer Resektion des defekten Kondylenkopfs;
- Fig. 8b: die Ansicht aus Fig. 8b, jedoch mit ausgeblendetem Cranium;
- Fig. 9a: ein Ergebnis des Anpassens der funktionalen Gruppe eines Kiefergelenksprothese-3D-Modells an die Schädelanatomie des Patienten von schräg rechts unten;

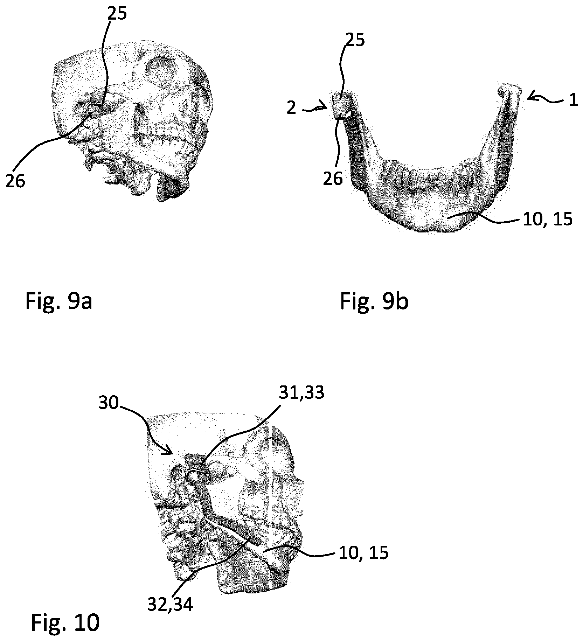

- Fig. 9a: die Situation aus Fig. 9a, jedoch von vorne aus gesehen bei ausgeblendetem Cranium;
- Fig. 10: ein Kiefergelenksprothese-3D-Modell, angeordnet an der graphischen Darstellung des Schädels des Patienten;

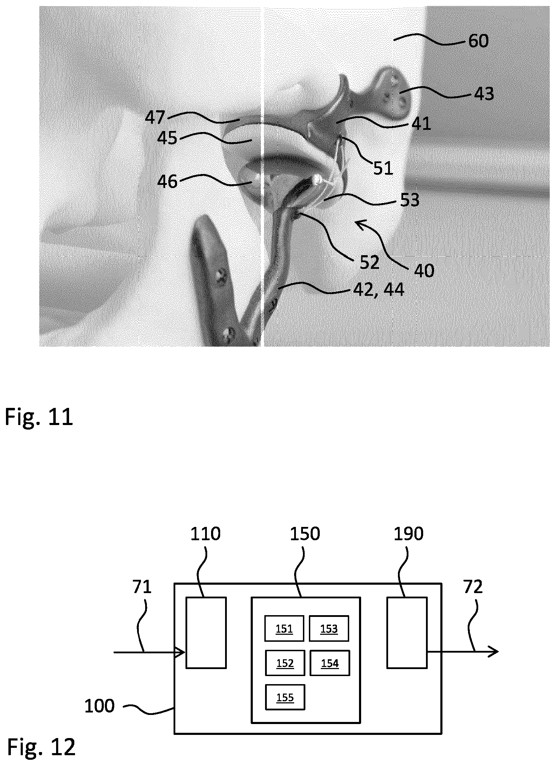

- Fig. 11: eine Kiefergelenksprothese, hergestellt nach einer Ausführungsform der vorliegenden Erfindung, angebracht an einem Schädelmodell;
- Fig. 12: eine schematische Darstellung eines Computersystems gemäß einer weiteren Ausführungsform der vorliegenden Erfindung;

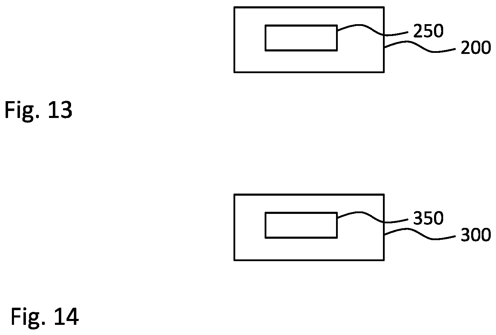

- Fig. 13: eine schematische Darstellung eines Computerprogrammprodukts gemäß einer weiteren Ausführungsform der vorliegenden Erfindung; und
- Fig. 14: eine schematische Darstellung eines Datenspeichermediums gemäß einer weiteren Ausführungsform der vorliegenden Erfindung.

In sämtlichen Figuren sind gleiche bzw. funktionsgleiche Elemente und Vorrichtungen - sofern nichts anderes angegeben ist - mit denselben Bezugszeichen versehen worden.

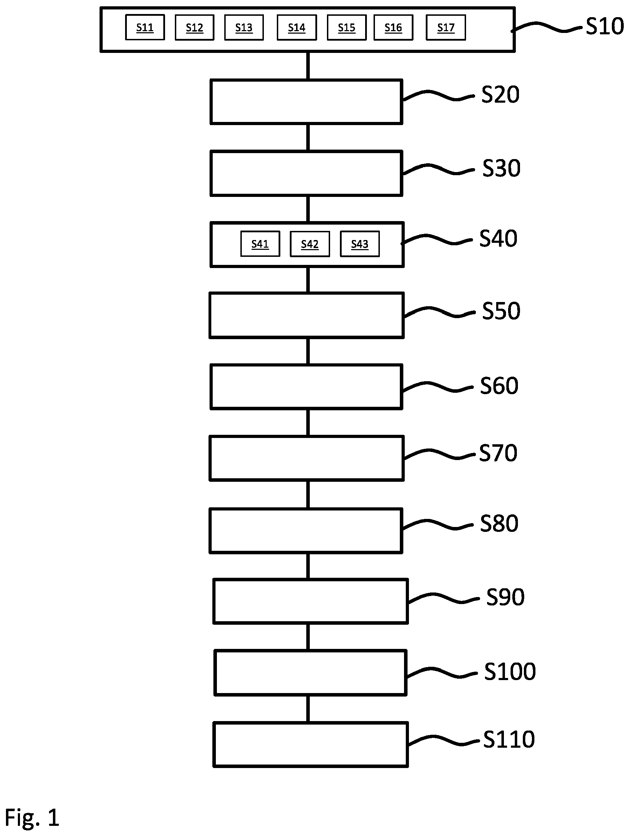

Fig. 1 zeigt ein schematisches Flussdiagramm zum Erläutern eines Verfahrens gemäß einer Ausführungsform der vorliegenden Erfindung.

In einem Schritt S10 wird eine 3-dimensionale (3D-) Darstellung des Schädels eines Patienten bereitgestellt, wobei die Darstellung zumindest die Kiefergelenke (oder was davon gegebenenfalls nach einem Unfall oder einer Krankheit verblieben ist) umfasst.

Hierfür können zunächst in einem Schritt S11 anatomische Daten des Schädels des Patienten bereitgestellt (ausgelesen, angefragt und empfangen, etc.) werden. Das Bereitstellen S11 der anatomischen Daten des Schädels des Patienten kann beispielsweise unter Verwendung von (oder durch Bereitstellen von) medizinischen Bildgebungsdaten erfolgen, d.h. insbesondere von Daten, welche durch medizinische Bildgebungsverfahren, insbesondere Tomographieverfahren wie etwa Computertomographie (CT), digitale Volumentomographie (DVT) bzw. engl. "cone beam computed tomography", CBCT, Magnetresonanztomographie (MRT) und dergleichen erzeugt wurden.

Solche medizinischen Bildgebungsdaten liegen für gewöhnlich im DICOM-Format vor, wobei auch andere Formate möglich sind. "DICOM" ist eine eingetragene Marke und steht für "Digital Imaging and Communications in Medicine". Wann immer hierin von dem DICOM-Standard die Rede ist, kann darunter insbesondere die aktuelle Version des DICOM-Standards PS3.1 2020d verstanden werden, wobei auch strukturierte Reports nach einer vorangehenden oder einem nachfolgenden DICOM-Standard-Version verwendet werden können. Die DICOM-Daten können insbesondere aus einem PACS ausgelesen oder angefragt werden, wobei PACS für "Picture Archiving and Communication System" steht.

Üblicherweise liegen die Bildgebungsdaten im DICOM-Format als Schichtbilder vor, aus denen durch eine entsprechende 3D-Bilderzeugungssoftware 3D-Daten erzeugt werden. Die 3D-Daten können beispielsweise im STL-Format vorliegen. Das STL-Format beschreibt die Oberfläche von 3D-Körpern mithilfe von Dreiecksfacetten, von denen jede durch die drei Eckpunkte und die zugehörige Flächennormale des Dreieckes charakterisiert wird.

Es versteht sich, dass die ursprünglichen medizinischen Bildgebungsdaten zusätzliche Körperteile und/oder Knochen außerhalb des Schädels des Patienten aufweisen können. Diese können bei den bereitgestellten anatomischen Daten des Schädels mit enthalten sein, oder in einem Zwischenschritt entfernt werden, da für das Entwerfen und spätere Herstellen der Kiefergelenksprothese die Daten über die Anatomie der Knochen des Schädels ausreichend sind.

In einem Schritt S12 kann eine Segmentierung, d.h. insbesondere eine Klassifizierung von Pixeln oder Voxeln der anatomischen Daten als jeweils zu einem bestimmten Knochen (oder anderen menschlichen Körperteil) gehörig, auf Grundlage der medizinischen Bildgebungsdaten durchgeführt werden. Hierfür eignen sich beispielsweise trainierte künstliche Neuronale Netze, insbesondere so genannte "convolutional neural networks" (CNNs). Auf Basis einer solchen Segmentierung können somit automatisch die für das vorliegende Verfahren notwendigen Bestandteile des menschlichen Skeletts, d.h. insbesondere der Schädel, erkannt und die relevanten Bildgebungsdaten, welche diese Bestandteile betreffen (d.h. umfassen) für die weitere Verwendung optional isoliert werden.

Die anatomischen Daten können auch aus medizinischen Bildgebungsdaten mehrerer Quellen zusammengesetzt werden. Beispielsweise können auch Dentalscans (oder: "Dentaldaten"), beispielsweise durch konventionelle oder 3D-Röntgenaufnahmen der Zähne des Patienten, Teil der 3D-Daten sein. Die Dentalscans können beispielsweise auf klassischen Gipsabdrücken der Zähne basieren, die entweder durch denselben CT-Scanner gelassen werden wie der Patient zuvor (dann liegen die Daten als DICOM Bilderstapel vor). Alternativ können Detalscans gegebenenfalls mit modernen 3D Oral-Scannern direkt im Mund des Patienten abgescannt werden. Im letzteren Fall liegen die Detalscans somit vorteilhaft gleich im STL-Format vor, und müssen nicht per Gipsabdruck/CT generiert werden.

Die relevanten Bildgebungsdaten können anschließend, in einem Schritt S13, zusammen mit der Segmentierung aus Schritt S12 in eine 3D-Orthognathie-Software eingelesen und darin bearbeitet werden. Die 3D-Orthognatie-Software kann wiederum aus den einzelnen Bildgebungsdaten (z.B. DICOM Daten wie etwa DICOM Schichtbildern) 3D-Darstellungen erzeugen und beispielsweise auf einem Monitor, welcher operativ mit der Rechenvorrichtung verbunden ist, dargestellt werden.

Das Bereitstellen S10 der graphischen Darstellung kann auch ein Aufbereiten der bereitgestellten medizinischen Bildgebungsdaten umfassen. Falls es erforderlich sein sollte, kann z.B. mittels der 3D-Orthognathie-Software eine komplette Orthognathie-Planung durchgeführt werden, um die Stellung von Ober- und Unterkiefer exakt zu definieren, und eine Zielokklusion abzubilden, wobei wie in im Vorangehenden beschrieben Dentaldaten verwendet werden können.

Der Begriff Okklusion bezeichnet jeglichen Kontakt der Zähne des Oberkiefers mit denen des Unterkiefers. Die Planung, oder jede andere Manipulation oder Aufbereitung der eingelesenen Bildgebungsdaten oder der erzeugten 3D-Daten kann mittels einer Eingabevorrichtung durchgeführt werden, die operativ mit einer Rechenvorrichtung, welche die Software ausführt, und einer Darstellungseinrichtung (z.B. Monitor) verbunden ist. Die Eingabevorrichtung kann beispielsweise eine Tastatur, eine Computermaus, ein haptisches Eingabegerät oder dergleichen umfassen. Die Eingabevorrichtung kann auch einen Touchscreen umfassen, sodass Eingabevorrichtung und Monitor auch ineinander integriert sein können, siehe auch Fig. 12 und die dazugehörige Beschreibung.

Aus dem Orthognathie-Programm können in einem Schritt S14 einzelne Datenstrukturen bzw. Elemente, insbesondere Schädel, Maxilla, Unterkiefer (Mandibula) etc., zusammen mit einem Final Splint wiederum im STL-Format exportiert werden.

In einem Schritt S15 können die verschiedenen STL-Daten aus der 3D-Bilderzeugungssoftware und/oder der 3D-Orthognatie-Software in eine graphische Benutzerschnittstelle zum Modellieren von 3D-Strukturen (oder :3D-Modellier-Software) eingelesen werden. In der graphischen Benutzerschnittstelle wird schließlich in einem Schritt S16 eine 3-dimensionale (3D-) Darstellung des Schädels des Patienten generiert, welche als Basis für das weitere Verfahren dient.

Alternativ zu den Schritten S11-S16, welche zum Bereitstellen S10 der 3D-Darstellung des Schädels des Patienten dienen, kann auch direkt eine etwaige bereits vorhandene 3D-Darstellung des Schädels durch Auslesen aus einem (flüchtigen oder nicht-flüchtigen) Datenspeicher bereitgestellt werden. Beispielsweise kann eine akkurate 3D-Darstellung bereits aus einem kürzlich erfolgten vorhergehenden Eingriff am Schädel des Patienten vorliegen.

Nach dem Bereitstellen S10 der anatomischen Daten liegt somit vorteilhaft eine graphische Darstellung des Schädels, zumindest im Bereich der beiden Kiefergelenke, vor, welche insbesondere das Os temporale und die Mandibula umfasst.

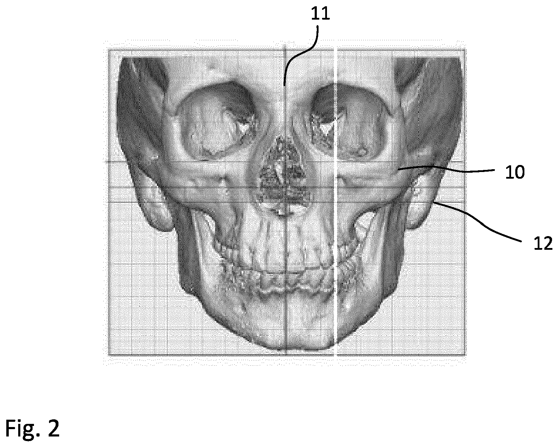

Fig. 2 zeigt schematisch eine solche 3-dimensionale (3D-) graphische Darstellung 10 des Schädels des Patienten.

In einem weiteren Schritt S17 kann in der graphischen Darstellung 10 automatisch eine vertikale Spiegelebene 11 (die Sagittalebene) und/oder eine Frankfurter Normale 12 eingefügt werden. Bei nicht vollständig spiegelsymmetrischen anatomischen Gegebenheiten in der 3D-Darstellung kann eine am besten passende Sagittalebene automatisch bestimmt werden, beispielsweise unter Verwendung einer Künstliche-Intelligenz-Entität wie etwa einer Support-Vector-Maschine. Eine solche automatisch, oder durch einen Techniker vorab, definierte Spiegelebene wird bevorzugt durch einen Arzt noch überprüft und ggfs. angepasst.

In einem Schritt S20 wird ein 3-dimensionales (3D-) Modell für zumindest eine funktionale Gruppe der Kiefergelenksprothese, umfassend zumindest ein Kondylenkopf des Kiefergelenks und eine Gleitfläche für den Kondylenkopf des Kiefergelenks, bereitgestellt, beispielsweise aus 3D-Datensätzen eingelesen. Solche Datensätze können beispielsweise in eine branchenüblichen CAD-Software vorliegen.

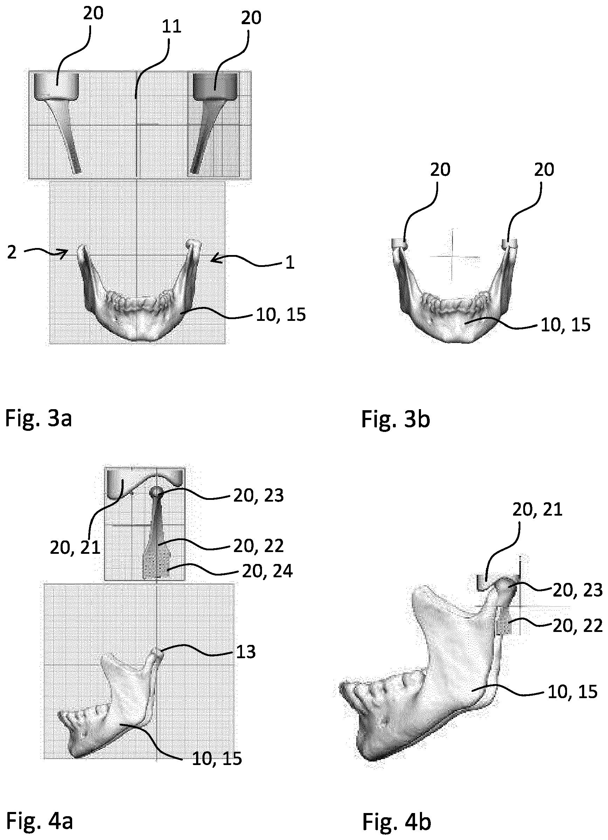

Fig. 3a und Fig. 4a zeigen jeweils im oberen Bereich ein solches Modell 20, wobei Fig. 3a eine Frontansicht und Fig. 4a eine Seitenansicht zeigt. In dem vorliegend verwendeten Beispiel ist die linke Seite des Patienten die gesunde Schädelseite, während die rechte Seite eine zu behandelnde Schädelseite 2 ist. Die gesunde Schädelseite wird hierin auch als Referenz-Schädelseite 1 bezeichnet, da sie im Regelfall für die Behandlung eine Referenz für eine funktionierende anatomische Anordnung bietet. Dargestellt ist jeweils lediglich eine graphische Darstellung des Unterkiefers 15, wobei insbesondere in Fig. 3a erkennbar ist, dass an der zu behandelnden Schädelseite 2 der Kondylenkopf des Unterkiefers 15 einen Defekt aufweist. Fig. 4a zeigt die Situation aus Fig. 3a, wobei auf Referenz-Schädelseite 1 geblickt wird.

Für das Modell 20 kann eine Vielzahl von Modellen verwendet werden, welche jeweils unterschiedliche Charakteristiken bezüglich Komplexität, Funktionalität und dergleichen aufweisen. Es hat sich überraschender Weise herausgestellt, dass bereits relativ einfach Funktionsmodelle für diese Zwecke gut geeignet sind. Hierbei können einige wenige elementare geometrische Objekte wie etwa Torusausschnitte, verwendet werden, um die funktionale Gruppe, aufgeteilt in eine Unterkieferkomponente 22 und eine als funktionales Gegenelement fungierende Craniumkomponente 21, zu beschrieben. Die Unterkieferkomponente 22 umfasst den - vorzugsweise grob spindelförmig gestalteten - Kondylenkopf 23 des 3D-Modells 20. Die Craniumkomponente 21 umfasst Fossa articularis und Tuberculum articulare, jeweils vorteilhaft als Torusausschnitte modelliert und miteinander kontinuierlich und stetig verbunden.

Für die konkrete Ausgestaltung und maßstäbliche Dimensionierung eines solchen Funktionsmodells können empirisch bestimmte Mittelwerte verwendet werden, um das Modell 20 zu bestimmen. Einmal bestimmt, kann das Modell 20 so für sämtliche Patienten als Grundlage dienen, wie im Weiteren detailliert beschrieben werden wird. In einem Schritt S30 wird das 3D-Modell 20 bezüglich der 3D-Darstellung 10 (d.h. in derselben graphischen Darstellung oder graphischen Umgebung, etwa einem virtuellen 3-dimensionalen Raum) angeordnet, und zwar vorteilhaft zunächst an der Referenz-Schädelseite 1. Die graphische Umgebung (welche auch als "graphische Benutzerschnittstelle", GUI, bezeichnet werden kann) kann beispielsweise mittels eines 2-dimensionalen Computerbildschirms dargestellt werden. Es ist aber auch denkbar, dass Virtual-Reality- oder Augmented-Reality-Vorrichtungen zum Einsatz kommen, etwa mit Hilfe von Virtual-Reality- oder Augmented-Reality-Brillen. Mithilfe solcher Vorrichtungen kann oft der 3-dimensionale Charakter einer 3-dimensionalen Darstellung objektiv besser dargestellt werden, da direkt das räumliche Vorstellungsvermögen eines Benutzers angesprochen bzw. ausgenutzt wird.

In einem Schritt S40 wird das 3D-Modell 20 daraufhin in der graphischen Umgebung vorteilhaft derart angepasst, d.h. insbesondere skaliert S41, positioniert S42 und/oder orientiert S43, dass das 3D-Modell 20 in bestimmungsgemäßer Funktions-Ausrichtung zu der graphischen Darstellung des Unterkiefers 15 steht. Mit anderen Worten wird das 3D-Modell 20 so angeordnet, wie das an der Referenzseite 1 befindliche Kiefergelenk positioniert und orientiert ist, wobei insbesondere auf den Kondylenkopf und die Fossa zu achten ist. Als Ziel kann formuliert werden, dass bis die Modell-Kontur des 3D-Modells 20 beidseitig, d.h. sowohl an Craniumkomponente 21 als auch Unterkieferkomponente 22, nahezu (d.h. im Wesentlichen) eben abschließt. Anders ausgedrückt kann das 3D-Modell 20 so angeordnet werden, dass der Kondylenkopf des 3D-Modells 20 mit der tatsächlichen Fossa an der Referenz-Schädelseite 1 interagieren (d.h. sich in dieser bewegen und/oder gleiten) könnte und/oder derart, dass der tatsächliche Kondylenkopf an der Referenz-Schädelseite 1 mit der Fossa des 3D-Modells 20 interagieren könnte.

Zum Anpassen S40 des 3D-Modells 20 kann die graphische Benutzerschnittstelle Funktionen aufweisen, die einem Benutzer das Drehen, Verschieben, Re-Skalieren etc. von Elementen der graphischen Darstellung 10 und/oder von Elementen (oder der Gesamtheit) des 3D-Modells 20 ermöglichen.

Fig. 3a und Fig. 4a zeigen jeweils die Situation nach Schritt S30. In der jeweils zugehörigen Fig. 3b bzw. Fig. 4b ist die Situation nach dem Durchführen von Schritt S40 gezeigt. Besonders in Fig. 4b ist gut ersichtlich, wie der Kondylenkopf 23 des 3D-Modells 20 den Kondylenkopf 13 (siehe Fig. 4a) an der Referenz-Schädelseite 1 der graphischen Darstellung des Unterkiefers 15 im Wesentlichen deckungsgleich überlagert.

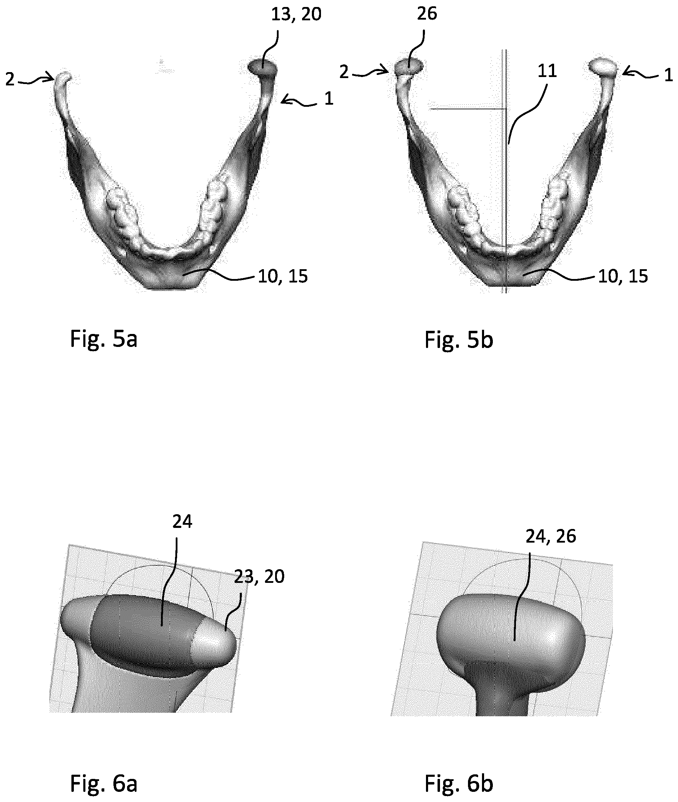

In einem Schritt S50 wird an der Spiegelebene 11 ein Kondylenkopf-Modell von der Referenz-Schädelseite an die zu behandelnde Schädelseite 2 gespiegelt. Fig. 5a zeigt hier in einer Ansicht auf den Unterkiefer 15 von oben, wie der Kondylenkopf 13 der 3D-Darstellung 10 zur Verwendung als Kondylenkopf-Modell 26 (siehe nachfolgende Beschreibung) an der Referenz-Schädelseite 1 markiert wird. Fig. 5b zeigt, wie in der 3D-Darstellung 10 nach dem Schritt S50 der defekte Kondylenkopf an der zu behandelnden Schädelseite 2 durch das Kondylenkopf-Modell 26 überlagert ist. Mittels und in der graphischen Benutzerumgebung kann der Benutzer nach dem Schritt S50 auch noch Nachkorrekturen vornehmen, um den das Kondylenkopf-Modell 26 an die Anatomie an der zu behandelnden Schädelseite 2 genau anzupassen, z.B. um sanfte Übergänge und bündige Abschlüsse zu erzielen.

Vorteilhaft wird als Kondylenkopf-Modell der Kondylenkopf 13 der Referenz-Schädelseite 1 der 3D-Darstellung 10 verwendet, sofern dieser Kondylenkopf 13 defektfrei ist. In diesem Fall kann davon ausgegangen werden, dass dieser Kondylenkopf 13 sich gut in die Anatomie des Patienten auch an der zu behandelnden Schädelseite 2 einfügt. Hierzu kann der Kondylenkopf 13, oder ein Teil davon, der Referenz-Schädelseite 1 der 3D-Darstellung 10 in der graphischen Umgebung von einem Benutzer zur Auswahl markiert werden.

Falls dies nicht möglich ist, beispielsweise falls der Kondylenkopf 13 auf der Referenz-Schädelseite einen Defekt aufweist oder eine Form aufweist, welche an der zu behandelnden Schädelseite 2 nicht verwendbar ist, kann als Kondylenkopf-Modell auch eine andere vordefinierte 3D-Struktur verwendet werden.

Beispielsweise kann alternativ als Kondylenkopf-Modell 26 auch, wie in Fig. 6a schematisch dargestellt ist, mindestens ein Teil des Kondylenkopfs 23 des 3D-Modells 20 hergenommen werden, etwa ein vorbestimmter Abschnitt 24 (oder: "Ausschnitt", oder "Teilbereich"), welcher zuvor bereits gemäß Schritt S40 skaliert wurde. Fig. 6b illustriert einen derart definierten, reskalierten, positionierten und orientierten Abschnitt 24, optional nach einer Kantenabrunden und/oder Detailanpassung an einen Abschnitt (z.B. eine Unterkieferfixationsplatte) einer Unterkieferkomponente der Kiefergelenksprothese.

Weiter alternativ zu dem Vorbeschriebenen kann auch eine andere vorbestimmte 3D-Struktur als Kondylenkopf-Modell 26 verwendet werden.

In einem Schritt S60 wird eine Oberflächengeometrie der Fossa von dem 3D-Modell 20 extrahiert, welche die spätere Fossa-Gleitfläche der Kiefergelenksprothese für den Kondylenkopf der Kiefergelenksprothese darstellen, oder zumindest als Basis für diese dienen, soll. In einigen Varianten kann das 3D-Modell 20 bereits derart konzipiert sein, dass die Fossa darin lediglich als 3D-Fläche ausgeführt ist, d.h. lediglich aus ihrer Fossa-Gleitfläche 25 besteht.

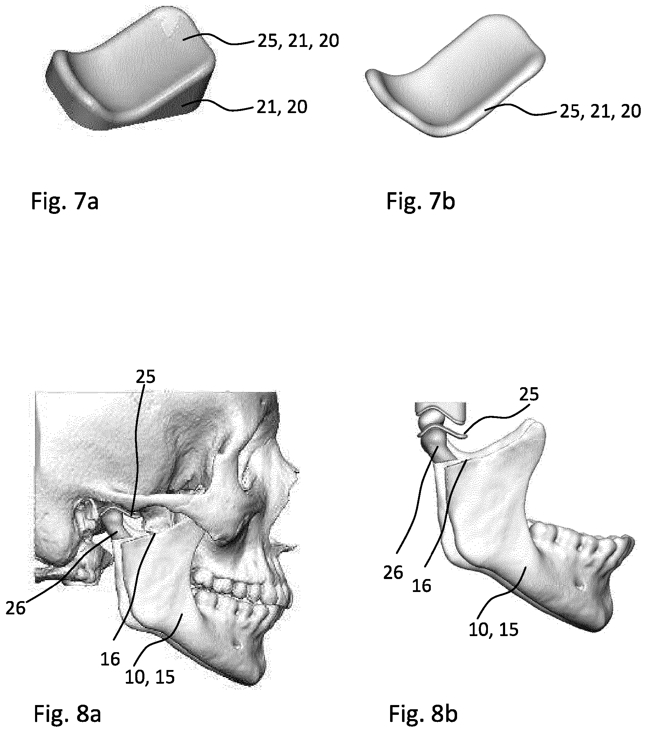

Fig. 7a zeigt einen Abschnitt der Craniumkomponente 21 des 3D-Modells 20 mit Blick auf die Fossa-Gleitfläche 25. Gut erkennbar ist die Höhlung in der Gleitfläche 25, in welche später der Kondylenkopf (bzw. dessen Substitut) der Kiefergelenksprothese eingreifen soll.

Fig. 7b zeigt die extrahierte Fossa-Gleitfläche 25 isoliert.

In einem Schritt S70 wird die extrahierte Fossa-Gleitfläche 25 an der zu behandelnden Schädelseite angeordnet. Dies kann beispielsweise, wie im Vorangehenden bereits mit Bezug auf den Kondylenkopf 23 beschrieben wurde, durch Spiegeln an der Spiegelebene 11 erfolgen. Dies hat den Vorteil, dass aufgrund der in Schritt S40 vorgenommenen Anpassung die Fossa-Gleitfläche 25 an der Referenz-Schädelseite 1 bereits passend ausgerichtet ist und somit davon ausgegangen werden kann, dass nach deren Spiegelung an der Spiegelebene 11 auch eine im Wesentlichen passende Ausrichtung der Fossa-Gleitfläche 25 an der zu behandelnden Schädelseite 2 vorliegt.

Alternativ kann die Fossa-Gleitfläche 25 auch direkt aus dem in Schritt S41 reskalierten 3D-Modell 20 extrahiert werden und daraufhin direkt an der zu behandelnden Schädelseite 2 angeordnet werden. Hiernach können in der Regel weitere Anpassungs-, Positionierungs- und/oder (Re-) Orientierungsschritte nötig sein, analog den im Vorangehenden beschriebenen Schritten S41, S42, S43.

In einem Schritt S80 werden das gespiegelte Kondylenkopf-Modell 26 sowie die Fossa-Gleitfläche 25 an der zu behandelnden Schädelseite 2 aneinander und/oder an eine Anatomie des Patienten auf der zu behandelnden Schädelseite 2 angepasst.

Bei einem medizinischen Eingriff kann der beschädigte Teil des Kiefergelenks des Patienten an der zu behandelnden Schädelseite 2 entfernt werden (Resektion). Diese sowie deren Ergebnis können in der graphischen Darstellung bereits geplant und berücksichtigt werden.

Fig. 8a zeigt die graphische Darstellung 10 des Schädels des Patienten von der zu behandelnden Schädelseite 2 nach erfolgter Entfernung der graphischen Darstellung defekten Kondylenkopfs 13. Die in Fig. 8a gezeigte graphische Darstellung 10 kann entweder das Resultat einer simulierten Resektion sein, d.h. einer bloß an der graphischen Darstellung 10 durchgeführten Operation. Alternativ kann die gezeigte graphische Darstellung 10 auch auf nach einer tatsächlichen Resektion durchgeführten medizinischen Bildgebungsverfahren basieren, z.B. auf einem oder mehreren Tomographieverfahren.

In Fig. 8a ist auch gut ersichtlich, wie in dem Schritt S80 die Fossa-Gleitfläche 25 und das Kondylenkopf-Modell 26 aneinander sowie an die Schädelanatomie angepasst werden können. Die graphische Benutzerschnittstelle kann das Ein- und Ausblenden verschiedener Teilbereiche der graphischen Darstellung 10 ermöglichen.

In Fig. 8b ist etwa der Fall gezeigt, in welchem der Großteil des Craniums ausgeblendet wurde. Somit kann, z.B. nach optimaler Anpassung (Anordnung, Orientierung, etc.) der Fossa-Gleitfläche 25 an die Anatomie des Cranium, bei ausgeblendetem Cranium die Anordnung, Orientierung etc. des Kondylenkopf-Modells 26 an die Fossa-Gleitfläche 25 auf der zu behandelnden Schädelseite 2 feinjustiert werden, insbesondere, wie in Fig. 8b gezeigt, bei gleichzeitiger Betrachtung, als Referenz, der Interaktion und Anordnung von Kondylenkopf 13 und zugehöriger Fossa-Gleitfläche auf der Referenz-Schädelseite 1. In Fig. 8a und Fig. 8b ist außerdem auch die Resektions-Berandung 16 zu sehen, welche, wie bereits erwähnt, entweder eine tatsächliche Resektions-Berandung oder aber eine virtuelle, lediglich geplante oder geschätzte Resektions-Berandung darstellen kann.

Fig. 9a und Fig. 9b zeigen ein Endergebnis der Anpassung in Schritt S80 aus zwei verschiedenen Perspektiven: Fig. 9a zeigt die funktionale Gruppe der geplanten Kiefergelenksprothese, nämlich die Fossa-Gleitfläche 25 und das Kondylenkopf-Modell 26, von schräg unten bei größtenteils eingeblendetem Cranium. Fig. 9b zeigt den Unterkiefer 15 mit ergänzter Fossa-Gleitfläche 25 und ergänztem Kondylenkopf-Modell 26 direkt von vorne, wobei der Großteil des Craniums ausgeblendet ist, und die Ansicht die funktionale Gruppe der geplanten Kiefergelenksprothese gemeinsam mit der funktionalen Gruppe des Kiefergelenks an der gesunden Referenz-Schädelseite 1 darstellt.

Beide Darstellungen, gemäß Fig. 9a und Fig. 9b, können in der graphischen Benutzerschnittstelle durch den Benutzer beliebig rotiert, vergrößert, verkleinert und in sonstiger Weise in der Anzeige verändert werden, sodass der Benutzer eine optimale Anpassung an die anatomischen Gegebenheiten des Patienten erzielen kann.

In einem Schritt S90 kann, basierend auf der in Schritt S80 angepassten funktionalen Gruppe aus Kondylenkopf-Modell 26 und Fossa-Gleitfläche 26, ein Kiefergelenksprothese-3D-Modell 30 erzeugt werden. Hierzu können insbesondere Übergänge zur nativen Knochensituation patientenspezifisch designt, werden und basierend auf einer Designrichtlinie (z.B. mit einer definierten Mindestdimensionierung) ausgelegt werden.

Fig. 10 zeigt beispielhaft eine solches erzeugtes Kiefergelenksprothese-3D-Modell 30, angeordnet an der graphischen Darstellung 10 des Schädels des Patienten genau derart, wie in realiter später die Kiefergelenksprothese selbst an dem tatsächlichen Schädel des Patienten angebracht werden soll.

Das Kiefergelenksprothese-3D-Modell 30 umfasst dabei eine Craniumkomponente 31 und eine Unterkieferkomponente 32. Die Craniumkomponente 31 umfasst die Fossa-Gleitfläche 25 sowie eine mit dieser verbundene Craniumfixationsplatte 33. Die Unterkieferkomponente 32 umfasst das einen Kondylenkopf gemäß dem Kondylenkopf-Modell 26 sowie eine Unterkieferfixationsplatte 34. In Fig. 10 ist die Unterkieferfixationsplatte 34 als langgezogener, verformter Streifen dargestellt. Je nach den konkreten anatomischen Gegebenheiten des Patienten kann die Unterkieferfixationsplatte 34 jedoch auch andere Formen aufweisen. Beispielsweise kann die Unterkieferfixationsplatte 34 zusätzlich zu ihrer langgestreckten Form entlang der Außenkontur des Unterkiefers auch noch einen oder mehrere Querausläufer aufweisen und/oder abgewinkelt sein.

Die Craniumkomponente 31 kann so ausgelegt sein, dass sie einen Fossa-Grundkörper aufweist, welcher die Fossa-Gleitfläche 26 trägt, wobei es der Fossa-Grundkörper ist, an welchem die Craniumfixationsplatte 33 befestigt ist oder entspringt.

Die Craniumfixationsplatte 33 weist vorzugsweise Öffnungen auf, in welche Befestigungsmittel, beispielsweise Schrauben, zur Befestigung der Craniumfixationsplatte 33 an dem Cranium des Patienten eingebracht werden können. Die Unterkieferfixationsplatte 34 weist vorzugsweise Öffnungen auf, in welche Befestigungsmittel, beispielsweise Schrauben, zur Befestigung der Unterkieferfixationsplatte 34 an dem Unterkiefer des Patienten eingebracht werden können.

Optional kann an der Craniumkomponente 31 und an der Unterkieferkomponente 32 jeweils eine Öse vorgesehen werden. Durch diese beiden Ösen kann ein Faden oder ein Band hindurchgeführt und verknotet werden, sodass der dann geschlossene Faden (oder dergleichen) die beiden Ösen und somit die Craniumkomponente 31 und die Unterkieferkomponente 32 miteinander beweglich koppelt, siehe hierzu auch Fig. 11 und die zugehörige Beschreibung.

Das Entwerfen des Kiefergelenksprothese-3D-Modells 30 derart, dass diese die genannten Ösen umfasst, erfolgt vorteilhaft als letzter Schritt im Erzeugen des Kiefergelenksprothese-3D-Modells 30, da zu diesem Zeitpunkt die zu erwartenden Bewegungen und Freiheitsgrade, welche die Kiefergelenksprothese erlauben wird, ersichtlich sind.

In einem Schritt S100 werden Konstruktionsdaten basierend auf dem erzeugten Kiefergelenksprothese-3D-Modell 30 erzeugt und ausgegeben. Beispielsweise können die Konstruktionsdaten ganz oder teilweise an eine oder mehrere Maschinen zur automatischen Herstellung einer Kiefergelenksprothese 40 gemäß dem Kiefergelenksprothese-3D-Modell 30 ausgegeben werden. Zusammen mit den Konstruktionsdaten können auch Steuersignale ausgegeben werden, welche die Maschinen entsprechend steuern.

In einem optionalen Schritt S110 kann die Kiefergelenksprothese nach Maßgabe der ausgegebenen Konstruktionsdaten, d.h. entsprechend dem Kiefergelenksprothese-3D-Modell 30 hergestellt werden, wie im Folgenden erläutert wird. In Ausführungsformen, in denen das Verfahren diesen Schritt S110 umfasst, kann das Verfahren auch als Verfahren zum Herstellen einer Kiefergelenksprothese bezeichnet werden. Selbstverständlich kann der Schritt S110 zum Herstellen der Kiefergelenksprothese basierend auf den Konstruktionsdaten auch unabhängig von den vorangehenden Schritten durchgeführt werden, insbesondere räumlich und/oder zeitlich von der Ausführung der vorangehenden Schritte getrennt.

In einem optionalen weiteren Schritt kann die Kiefergelenksprothese schließlich in einen Patienten eingesetzt werden.

Fig. 11 zeigt eine beispielhafte Darstellung, für die eine Kiefergelenksprothese 40, welche gemäß der vorliegenden Erfindung ausgelegt und hergestellt wurde, zur Veranschaulichung auf einem Schädelmodell 60 befestigt wurde.

Fig. 11 zeigt dabei, wie die Kiefergelenksprothese 40 (ganz analog zu dem zugrundeliegenden Kiefergelenksprothese-3D-Modell 30) eine Craniumkomponente 41 und eine Unterkieferkomponente 42 aufweist.

Die Craniumkomponente 41 umfasst eine Craniumfixationsplatte 43, welche in der in Fig. 11 gezeigten Situation mit Schrauben an dem Schädelmodell 60 angeschraubt wurde. Die Craniumkomponente 41 umfasst außerdem einen Fossa-Grundkörper 47, welcher mit der Craniumfixationsplatte 43 verbunden ist und dazu ausgelegt ist, die Fossa-Gleitfläche 45 zu tragen. Craniumfixationsplatte 43 und Fossa-Grundkörper 47 können vorteilhaft einstückig ausgebildet werden, insbesondere generativ (oder, anders ausgedrückt: additiv), bevorzugt in einem Verfahren zum selektiven Laserschmelzen von Titan.

Die Fossa-Gleitfläche 45 hingegen wird vorteilhaft aus einem Polyethylen-Material ausgebildet, bevorzugt aus "ultra-high-molecular-weight polyethylene" (abgekürzt mit "UHMWPE" oder teilweise auch mit "UHMW") UHMWPE ist ein thermoplastischer Polyethylen mit ultra-hohem Molekülgewicht, beispielsweise von 2 bis 8 Millionen g/mol. Bevorzugt wird die Fossa-Gleitfläche 45 mit Hilfe eines Sinterprozesses mit dem aus Titan gefertigten Fossa-Grundkörper verbunden. Somit kann die Craniumkomponente 41 insgesamt aus einem Verbundwerkstoff (oder: Kompositwerkstoff) aus Titan und UHMWPE ausgebildet sein. Der aus UHMWPE gebildete Bestandteil kann beispielsweise als eigenständiger Bestandteil aufgeschmolzen werden, mit nur einer Fläche als Haftgrund. Eine den UHMWPE-Bestandteil umgebende Wandung im Stile eines Inlays ist somit zwar möglich, aber nicht erforderlich.

Die Unterkieferkomponente 42 umfasst neben dem Kondylenkopf 46, welcher genau entsprechend dem Kondylenkopf-Modell 26 in dem Kiefergelenksprothese-3D-Modell 30 ausgebildet ist, die Unterkieferfixationsplatte 44. In dem in Fig. 11 gezeigten Beispiel weist die Unterkieferfixationsplatte einen abgewinkelten Abschnitt auf, welcher sich in Richtung des Kronenfortsatzes des Unterkiefers erstreckt.

Die Unterkieferkomponente 42 wird bevorzugt einstückig hergestellt, insbesondere generativ (oder, anders ausgedrückt: additiv), besonders bevorzugt in einem Verfahren zum selektiven Laserschmelzen von Titan. Zur Verbesserung der Gleitfähigkeit des Kondylenkopfs 46 an der Fossa-Gleitfläche 45, d.h. um die Artikulationspaarung möglichst reibungsarm zu gestalten, wird der Kondylenkopf 46 vorteilhaft poliert. Somit besteht die Gleitpaarung zwischen den im Grunde separaten Teil-Implantaten der Craniumkomponente 41 und der Unterkieferkomponente 42 bevorzugt aus UHMWPE und poliertem Titan.

Es ist aber auch denkbar, die Unterkieferkomponente 42 ebenfalls als Kompositbauteil herzustellen, wobei insbesondere der Kondylenkopf 46 aus einem anderen Material ausgebildet sein kann als der Rest der Unterkieferkomponente 42, insbesondere die Unterkieferfixationsplatte 44. Beispielsweise kann der Kondylenkopf 46 aus einem Keramikwerkstoff ausgebildet sein.

Fig. 11 zeigt außerdem auch eine an der Craniumkomponente 41 angebrachte Craniumkomponentenöse 51 sowie eine an der Unterkieferkomponente 42 angebrachte Unterkieferkomponentenöse 52. Außerdem ist ein ringförmig geschlossener, die Craniumkomponentenöse 51 und die Unterkieferkomponentenöse 52 koppelnder Faden 53 dargestellt.

Der Faden 53 kann vor oder nach dem Einsetzen der Kiefergelenksprothese 40 an dem Patienten an den Ösen 51, 52 angebracht werden. Aufgrund der komplexen Bewegungsfunktionen des Kiefergelenks und der mitunter leicht unterschiedlichen Funktionen der Kiefergelenksprothese 40 kann der Faden 53 dem Patienten anfänglich eine Hilfestellung bieten, bis das Gehirn des Patienten die neue Situation verarbeitet hat. Insbesondere kann der Faden 53 verhindern, dass der Patient Craniumkomponente 41 und Unterkieferkomponente 42 zu weit voneinander entfernt, d.h. der Faden 53 kann eine Haltefunktion ausfüllen oder, anders ausgedrückt, Referenz-Bewegungen erlauben.

Der Faden 53 (oder das Band oder dergleichen) kann als vorteilhaft aus einem resorbierbaren Material ausgebildet sein. Die Resorptionsdauer des Materials wird vorteilhaft derart bemessen, dass der Faden 53 zu einem Zeitpunkt vollständig resorbiert ist, zu welchem bei der Mehrzahl der Fälle dessen Funktion nicht mehr benötigt wird. Wenn der Heilungsprozess nämlich fortgeschritten ist, und dem Patienten die neuen Positionen und Bewegungsabläufe vergegenwärtigt sind und natürlich erscheinen, werden Ober- und Unterkiefer im Regelfall wieder durch die natürliche Muskelschlinge gehalten und kontrolliert, wie dies auch in der nativen/gesunden Situation der Fall ist. Dies fügt sich in die gesamte Idee hinter der vorliegenden Erfindung ein, wonach die Kiefergelenksprothese 40 so nah an den natürlichen anatomischen Gegebenheiten und Funktionalitäten nicht nur eines Durchschnittspatienten, sondern ganz spezifisch eines jeden individuellen Patienten, sein soll wie möglich.

Fig. 12 zeigt eine schematische Darstellung eines Computersystem 100 gemäß einer weiteren Ausführungsform der vorliegenden Erfindung. Das Computersystem 100 ist dazu ausgelegt und eingerichtet, das erfindungsgemäße Verfahren zum Entwerfen einer Kiefergelenksprothese 40 durchzuführen, bevorzugt das im Vorangehenden mit Bezug auf die Fig. 1 bis Fig. 11 beschriebene Verfahren. Insbesondere kann das Computersystem 100 hierzu aus einem Personal Computer, PC, bestehen, auf welchem eine entsprechende Software ausgeführt wird. Die Software kann dazu ausgelegt sein, dem Benutzer das Durchführen der im Vorangehenden beschriebenen Schritte S10 bis S100 zu ermöglichen.

Das Computersystem 100 kann eine Eingabeschnittstelle 110 umfassen, mittels welcher medizinische Daten 71 eines Patienten einlesbar sind. Bei den medizinischen Daten 71 kann es sich um medizinische Bilddaten, bearbeitete Daten und/oder dergleichen handeln, wie dies im Vorangehenden insbesondere mit Bezug auf Schritt S10 beschrieben wurde. Insbesondere können die medizinischen Daten 71 DICOM-Daten umfassen, etwa Tomographie-Bilddaten, Dentaldaten und dergleichen. Die medizinischen Daten 71 können beispielsweise von einem PACS (engl. "picture archiving and communication system"), von einer Cloud-Speicherlösung o.ä. empfangen und/oder angefragt werden.

Von der Eingabeschnittstelle 110 können die empfangenen medizinischen Daten 71 an eine Recheneinrichtung 150 übermittelt werden, welche üblicherweise eine zentrale Prozessoreinheit 151 (engl. "central processing unit", CPU), einen Arbeitsspeicher 152 (engl. "random access memory", RAM), einen nicht-flüchtigen Datenspeicher 153, eine Darstellungseinrichtung 154 (z.B. Monitor, AR- oder VR-Brille oder Touchscreen) und eine Benutzereingabeschnittstelle 155 (z.B. Tastatur und Computermaus, Touchscreen etc.) aufweist, die miteinander operativ zum Ausführen der Software und Durchführen des Verfahrens gekoppelt sind. Die Software kann hierbei in dem nicht-flüchtigen Datenspeicher 153 gespeichert und durch die zentrale Prozessoreinheit 151 in dem Arbeitsspeicher 152 ausgeführt werden. Alternativ oder zusätzlich kann die Software, oder es können einzelne Module oder Plug-Ins der Software, auch als Web-Service bereitgestellt werden.

Das Computersystem 100 kann außerdem eine Ausgabeschnittstelle 190 umfassen, welche dazu ausgelegt ist, die von der Recheneinrichtung 150 in dem Verfahren in Schritt S100 erzeugten Konstruktionsdaten 72 auszugeben, beispielsweise an einen Daten-Zwischenspeicher, an eine Maschine zum Herstellen eines oder mehrerer Teile der Kiefergelenksprothese 40 wie z.B. eine Maschine zum Erzeugen von Implantatteilen unter Verwendung von Laserschmelzen oder dergleichen mehr.

Fig. 13 zeigt eine schematische Darstellung eines Computerprogrammprodukts 200, welches ausführbaren Programmcode 250 umfasst, welcher dazu ausgelegt ist, wenn er ausgeführt wird, ein Verfahren gemäß einer Ausführungsform des Verfahrens zum Entwerfen einer Kiefergelenksprothese 40 durchzuführen, insbesondere das Verfahren, welches anhand von Fig. 1 bis 11 im Vorangehenden beschrieben wurde.

Fig. 14 zeigt eine schematische Darstellung eines nicht-flüchtigen, computerlesbaren Datenspeichermediums 300, welches ausführbaren Programmcode 350 umfasst, welcher dazu ausgelegt ist, wenn er ausgeführt wird, ein Verfahren gemäß einer Ausführungsform des Verfahrens zum Entwerfen einer Kiefergelenksprothese 40 durchzuführen, insbesondere das Verfahren, welches anhand von Fig. 1 bis 11 im Vorangehenden beschrieben wurde.

Zusammenfassend stellt die Erfindung ein Verfahren sowie ein Computersystem zum Entwerfen einer Kiefergelenksprothese für eine zu behandelnde Schädelseite eines Patienten bereit. Ein Kerngedanke des Verfahrens ist es, ein standardisiertes Modell des Kiefergelenks an einer graphischen Darstellung der gesunden Schädelseite des Patienten auszurichten, und dieses dann an einer Spiegelebene zu einer graphischen Darstellung der zu behandelnden Schädelseite hin zu spiegeln, um dort noch Detailanpassungen an dem Modell vorzunehmen.

### Bezugszeichenliste

- 1: Referenz-Schädelseite
- 2: zu behandelnde Schädelseite
- 10: graphische Darstellung des Schädels
- 11: Spiegelebene
- 12: Frankfurter Horizontale
- 13: Kondylenkopf der graphischen Darstellung des Unterkiefers
- 15: graphische Darstellung des Unterkiefers
- 16: Resektions-Berandung
- 20: 3D-Modell
- 21: Craniumkomponente des 3D-Modells
- 22: Unterkieferkomponente des 3D-Modells
- 23: Kondylenkopf des 3D-Modells
- 24: Abschnitt des Kondylenkopfs des 3D-Modells
- 25: Gleitfläche der Fossa des 3D-Modells
- 26: Kondylenkopf-Modell
- 30: Kiefergelenksprothese-3D-Modell
- 31: Craniumkomponente des Kiefergelenksprothese-3D-Modells
- 32: Unterkieferkomponente des Kiefergelenksprothese-3D-Modells
- 33: Craniumfixationsplatte des Kiefergelenksprothese-3D-Modells
- 34: Unterkieferfixationsplatte des Kiefergelenksprothese-3D-Modells
- 40: Kiefergelenksprothese
- 41: Craniumkomponente der Kiefergelenksprothese
- 42: Unterkieferkomponente der Kiefergelenksprothese
- 43: Craniumfixationsplatte der Kiefergelenksprothese
- 44: Unterkieferfixationsplatte der Kiefergelenksprothese
- 45: Fossa-Gleitfläche der Kiefergelenksprothese
- 46: Kondylenkopf der Kiefergelenksprothese
- 47: Fossa-Grundkörper der Kiefergelenksprothese
- 51: Craniumkomponentenöse
- 52: Unterkieferkomponentenöse
- 53: Faden
- 60: Schädelmodell
- 71: Medizinische Daten
- 72: Konstruktionsdaten
- 100: Computersystem
- 110: Eingabeschnittstelle
- 150: Recheneinrichtung
- 151: zentrale Prozessoreinheit
- 152: Arbeitsspeicher
- 153: nicht-flüchtiger Datenspeicher
- 154: Darstellungseinrichtung
- 155: Benutzereingabeschnittstelle
- 190: Ausgabeschnittstelle

- S10..S110: Verfahrensschritte

## Patentansprüche

1. Computerimplementiertes Verfahren zum Entwerfen einer Kiefergelenksprothese (40) für eine zu behandelnde Schädelseite (2) eines Patienten, mit den Schritten:
Bereitstellen (S10) einer 3-dimensionalen, 3D-, Darstellung (10) des Schädels umfassend zumindest die Kiefergelenke des Patienten;
Bereitstellen (S20) eines 3-dimensionalen, 3D-, Modells (20) für zumindest eine funktionale Gruppe der Kiefergelenksprothese (40), wobei die funktionale Gruppe zumindest einen Kondylenkopf (23) und eine Fossa-Gleitfläche (25) für den Kondylenkopf (23) umfasst;
Anordnen (S30) des bereitgestellten 3D-Modells (20) bezüglich der 3D-Darstellung (10) des Schädels an einer Referenz-Schädelseite (1) der 3D-Darstellung (10), wobei die Referenz-Schädelseite (1) eine der zu behandelnden Schädelseite (2) gegenüberliegende Schädelseite ist;
Anpassen (S40) des bereitgestellten 3D-Modells (20) in Größe und Ausrichtung an die anatomischen Verhältnisse der 3D-Darstellung (10) an der Referenz-Schädelseite (1);
Spiegeln (S50) eines Kondylenkopf-Modells (26) von der Referenz-Schädelseite (1) an die zu behandelnde Schädelseite (2), wobei das Kondylenkopf-Modell (26) entweder durch einen Kondylenkopf (13) der 3D-Darstellung (10) auf der Referenz-Schädelseite (1) oder durch mindestens einen Teil (24) des Kondylenkopfs (23) des 3D-Modells (20) gebildet wird;
Extrahieren (S60) einer Fossa-Gleitfläche (25) aus dem angepassten 3D-Modell (25);
Anordnen (S70) der extrahierten Fossa-Gleitfläche (25) an der zu behandelnden Schädelseite (2);
Anpassen (S80) des gespiegelten Kondylenkopf-Modells (26) sowie der Fossa-Gleitfläche (25) aneinander und/oder an eine Anatomie der 3D-Darstellung (10) auf der zu behandelnden Schädelseite (2); und
Ausgeben (S100) von Konstruktionsdaten (72) für die Kiefergelenksprothese (40), welche zumindest das angepasste gespiegelte Kondylenkopf-Modell (26) sowie die angepasste Fossa-Gleitfläche (25) umfassen.

2. Computerimplementiertes Verfahren nach Anspruch 1, umfassend:
Entwerfen eines Fossa-Grundkörpers (47) der Kiefergelenksprothese (40) derart, dass der Fossa-Grundkörper (47) die Fossa-Gleitfläche (45) trägt.

3. Computerimplementiertes Verfahren nach einem der Ansprüche 1 oder 2,
wobei das angepasste gespiegelte Kondylenkopf-Modell (26) als Teil einer separaten Craniumkomponente (41) der Kiefergelenksprothese (40) entworfen wird und die angepasste Fossa-Gleitfläche (45) der Kiefergelenksprothese (40) als Teil einer separaten Unterkieferkomponente (42) der Kiefergelenksprothese (40) entworfen wird.

4. Computerimplementiertes Verfahren nach Anspruch 3,
wobei die Craniumkomponente (41) und die Unterkieferkomponente (42) jeweils derart entworfen werfen, dass sie eine jeweilige Öse (51, 52) umfassen, mittels welcher die Craniumkomponente (41) und die Unterkieferkomponente (42) durch einen Faden (53) oder ein Band miteinander koppelbar sind.

5. Verfahren zur Herstellung einer Kiefergelenksprothese, umfassend:
Herstellen (S110) der Kiefergelenksprothese (40) nach Maßgabe der durch das computerimplementierte Verfahren gemäß einem der Ansprüche 1 bis 4 ausgegebenen Konstruktionsdaten (72).

6. Verfahren zur Herstellung einer Kiefergelenksprothese nach Anspruch 5,
wobei zumindest Teile (42, 43, 44, 46, 47) der Kiefergelenksprothese (40) mittels eines generativen Verfahrens unter Verwendung von Laserschmelzen erzeugt werden.

7. Verfahren zur Herstellung einer Kiefergelenksprothese nach Anspruch 5 oder 6,
wobei die Fossa-Gleitfläche (45) der Kiefergelenksprothese (40) als Gleitschicht aus einem Kunststoff, insbesondere aus einem Polyethylen, ausgebildet wird.

8. Verfahren zur Herstellung einer Kiefergelenksprothese nach einem der Ansprüche 5 bis 7,
wobei die Unterkieferkomponente (42) außer einem Kondylenkopf (46) gemäß dem Kondylenkopf-Modell (26) zumindest eine Unterkieferfixationsplatte (44) zum Fixieren der Unterkieferkomponente (42) am Unterkiefer des Patienten aufweist; und
wobei die Unterkieferkomponente (42) einstückig aus Titan gefertigt wird.

9. Verfahren zur Herstellung einer Kiefergelenksprothese nach einem der Ansprüche 5 bis 8,
wobei die Craniumkomponente (41) aus einem Verbund von Titan und einem Polyethylen ausgebildet wird und eine Craniumfixationsplatte (43) zum Fixieren der Craniumkomponente (41) am Cranium des Patienten aufweist, welche aus Titan geformt wird.

10. Computersystem (100), welches eingerichtet ist zum Durchführen des Verfahrens gemäß einem der Patentansprüche 1 bis 4.

11. Computerprogrammprodukt, welches einen Programmcode umfasst, welcher dazu ausgelegt ist, wenn er ausgeführt wird, das computerimplementierte Verfahren gemäß einem der Ansprüche 1 bis 4 auszuführen.

12. Computerlesbares, nicht-flüchtiges Datenspeichermedium, welches einen Programmcode umfasst, welcher dazu ausgelegt ist, wenn er ausgeführt wird, das computerimplementierte Verfahren gemäß einem der Ansprüche 1 bis 4 auszuführen.

13. Datenstream, welcher einen Programmcode umfasst, welcher dazu ausgelegt ist, wenn er ausgeführt wird, das computerimplementierte Verfahren gemäß einem der Ansprüche 1 bis 4 auszuführen.

14. Webserver, welcher dazu ausgelegt ist, das computerimplementierte Verfahren gemäß einem der Ansprüche 1 bis 4 als Webservice bereitzustellen.

## Claims

1. Computer-implemented method for designing a temporomandibular joint prosthesis (40) for a skull side (2) to be treated of a patient, comprising the steps of:
providing (S10) a 3-dimensional, 3D, representation (10) of the skull comprising at least the temporomandibular joints of the patient;
providing (S20) a 3-dimensional, 3D, model (20) for at least one functional group of the temporomandibular joint prosthesis (40), the functional group comprising at least a condylar head (23) and a fossa sliding surface (25) for the condylar head (23);
arranging (S30) the provided 3D model (20) with respect to the 3D representation (10) of the skull on a reference skull side (1) of the 3D representation (10), the reference skull side (1) being a skull side opposite the skull side (2) to be treated;
adapting (S40) the provided 3D model (20) in size and orientation to the anatomical conditions of the 3D representation (10) on the reference skull side (1);
mirroring (S50) a condylar head model (26) from the reference skull side (1) to the skull side (2) to be treated, the condylar head model (26) being formed either by a condylar head (13) of the 3D representation (10) on the reference skull side (1) or by at least one part (24) of the condylar head (23) of the 3D model (20);
extracting (S60) a fossa sliding surface (25) from the adapted 3D model (25);
arranging (S70) the extracted fossa sliding surface (25) on the skull side (2) to be treated;
adapting (S80) the mirrored condylar head model (26) and the fossa sliding surface (25) to one another and/or to an anatomy of the 3D representation (10) on the skull side (2) to be treated; and
outputting (S100) construction data (72) for the temporomandibular joint prosthesis (40) which comprise at least the adapted mirrored condylar head model (26) and the adapted fossa sliding surface (25).

2. Computer-implemented method according to claim 1, comprising:
designing a fossa base body (47) of the temporomandibular joint prosthesis (40) in such a way that the fossa base body (47) supports the fossa sliding surface (45).

3. Computer-implemented method according to either claim 1 or claim 2,
wherein the adapted mirrored condylar head model (26) is designed as part of a separate cranium component (41) of the temporomandibular joint prosthesis (40), and the adapted fossa sliding surface (45) of the temporomandibular joint prosthesis (40) is designed as part of a separate mandibular component (42) of the temporomandibular joint prosthesis (40).

4. Computer-implemented method according to claim 3,
wherein the cranium component (41) and the mandibular component (42) are each designed comprising an associated eyelet (51, 52), by means of which the cranium component (41) and the mandibular component (42) can be coupled to one another by a thread (53) or a strap.

5. Method for manufacturing a temporomandibular joint prosthesis, comprising: manufacturing (S110) the temporomandibular joint prosthesis (40) on the basis of the construction data (72) outputted by the computer-implemented method according to any of claims 1 to 4.

6. Method for manufacturing a temporomandibular joint prosthesis according to claim 5,
wherein at least parts (42, 43, 44, 46, 47) of the temporomandibular joint prosthesis (40) are manufactured by a generative process using laser melting.

7. Method for manufacturing a temporomandibular joint prosthesis according to either claim 5 or claim 6,
wherein the fossa sliding surface (45) of the temporomandibular joint prosthesis (40) is formed as a sliding layer made of a plastics material, in particular of a polyethylene.

8. Method for manufacturing a temporomandibular joint prosthesis according to any of claims 5 to 7,
wherein the mandibular component (42) has, in addition to a condylar head (46) according to the condylar head model (26), at least one mandibular fixation plate (44) for fixing the mandibular component (42) to the patient's mandible; and
wherein the mandibular component (42) is manufactured in one piece from titanium.

9. Method for manufacturing a temporomandibular joint prosthesis according to any of claims 5 to 8,
wherein the cranium component (41) is formed from a composite of titanium and a polyethylene and has a cranium fixation plate (43) formed from titanium for fixing the cranium component (41) to the patient's cranium.

10. Computer system (100) configured to carry out the method according to any of claims 1 to 4.

11. Computer program product, comprising program code configured to carry out, when executed, the computer-implemented method according to any of claims 1 to 4.

12. Computer-readable, non-volatile data storage medium, comprising program code configured to carry out, when executed, the computer-implemented method according to any of claims 1 to 4.

13. Data stream, comprising program code configured to carry out, when executed, the computer-implemented method according to any of claims 1 to 4.

14. Web server, configured to provide the computer-implemented method according to any of claims 1 to 4 as a web service.

## Revendications

1. Procédé mis en œuvre par ordinateur pour concevoir une prothèse d'articulation temporo-mandibulaire (40) pour un côté crânien à traiter (2) d'un patient, comportant les étapes consistant à :
fournir (S10) une représentation tridimensionnelle, 3D, (10) du crâne comprenant au moins les articulations temporo-mandibulaires du patient ;
fournir (S20) un modèle tridimensionnel, 3D, (20) pour au moins un groupe fonctionnel de la prothèse d'articulation temporo-mandibulaire (40), dans lequel le groupe fonctionnel comprend au moins une tête de condyle (23) et une surface de glissement de fosse (25) pour la tête de condyle (23) ;
disposer (S30) le modèle 3D (20) fourni par rapport à la représentation 3D (10) du crâne sur un côté crânien de référence (1) de la représentation 3D (10), dans lequel le côté crânien de référence (1) est un côté crânien opposé au côté crânien à traiter (2) ;
adapter (S40) le modèle 3D (20) fourni en termes de taille et d'alignement aux conditions anatomiques de la représentation 3D (10) sur le côté crânien de référence (1) ;
reproduire une image miroir (S50) d'un modèle de tête de condyle (26) du côté crânien de référence (1) sur le côté crânien à traiter (2), dans lequel le modèle de tête de condyle (26) est formé soit par une tête de condyle (13) de la représentation 3D (10) du côté crânien de référence (1), soit par au moins une partie (24) de la tête de condyle (23) du modèle 3D (20) ;
extraire (S60) une surface de glissement de fosse (25) à partir du modèle 3D (25) adapté ;
disposer (S70) la surface de glissement de fosse (25) extraite sur le côté crânien à traiter (2) ;
adapter (S80) le modèle de tête de condyle (26) reproduit en miroir ainsi que la surface de glissement de fosse (25) l'un à l'autre et/ou à une anatomie de la représentation 3D (10) sur le côté crânien à traiter (2) ; et
sortir (S100) des données de construction (72) pour la prothèse d'articulation temporo-mandibulaire (40), qui comprennent au moins le modèle de tête de condyle (26) reproduit en miroir, adapté, ainsi que la surface de glissement de fosse (25) adaptée.

2. Procédé mis en œuvre par ordinateur selon la revendication 1, comprenant de : concevoir un corps de base de fosse (47) de la prothèse d'articulation temporo-mandibulaire (40) de telle sorte que le corps de base de fosse (47) supporte la surface de glissement de fosse (45).

3. Procédé mis en œuvre par ordinateur selon l'une des revendications 1 ou 2, dans lequel le modèle de tête de condyle (26) reproduit en miroir, adapté, est conçu comme une partie d'un composant crânien séparé (41) de la prothèse d'articulation temporo-mandibulaire (40), et la surface de glissement de fosse (45) adaptée de la prothèse d'articulation temporo-mandibulaire (40) est conçue comme une partie d'un composant mandibulaire (42) séparé de la prothèse d'articulation temporo-mandibulaire (40).

4. Procédé mis en œuvre par ordinateur selon la revendication 3,
dans lequel le composant crânien (41) et le composant mandibulaire (42) ont été respectivement conçus de telle sorte qu'ils comprennent un œillet (51, 52) respectif au moyen duquel le composant crânien (41) et le composant mandibulaire (42) peuvent être couplés l'un à l'autre par une suture (53) ou une bande.

5. Procédure de fabrication d'une prothèse d'articulation temporo-mandibulaire, comprenant de :
fabriquer (S110) la prothèse d'articulation temporo-mandibulaire (40) sur la base des données de construction (72) sorties par le procédé mis en œuvre par ordinateur selon l'une des revendications 1 à 4.

6. Procédé de fabrication d'une prothèse d'articulation temporo-mandibulaire selon la revendication 5,
dans lequel au moins des parties (42, 43, 44, 46, 47) de la prothèse d'articulation temporo-mandibulaire (40) sont générées au moyen d'un procédé génératif utilisant une fusion au laser.

7. Procédé de fabrication d'une prothèse d'articulation temporo-mandibulaire selon la revendication 5 ou 6,
dans lequel la surface de glissement de fosse (45) de la prothèse d'articulation temporo-mandibulaire (40) est formée comme une couche de glissement constituée d'une matière plastique, en particulier d'un polyéthylène.

8. Procédé de fabrication d'une prothèse d'articulation temporo-mandibulaire selon l'une des revendications 5 à 7,
dans lequel le composant mandibulaire (42) comporte, en plus d'une tête de condyle (46) d'après le modèle de tête de condyle (26), au moins une plaque de fixation mandibulaire (44) pour fixer le composant mandibulaire (42) sur la mandibule du patient ; et
dans lequel le composant mandibulaire (42) est fabriqué d'un seul tenant à partir de titane.

9. Procédé de fabrication d'une prothèse d'articulation temporo-mandibulaire selon l'une des revendications 5 à 8,
dans lequel le composant crânien (41) est formé à partir d'un composite de titane et d'un polyéthylène et comporte une plaque de fixation crânienne (43) pour fixer le composant crânien (41) sur le crâne du patient, cette dernière étant formée à partir de titane.

10. Système informatique (100) configuré pour mettre en œuvre le procédé selon l'une des revendications 1 à 4.

11. Produit de programme informatique comprenant un code de programme qui est conçu pour, lorsqu'il est exécuté, réaliser le procédé mis en œuvre par ordinateur selon l'une des revendications 1 à 4.

12. Support de stockage de données non volatile lisible par ordinateur, comprenant un code de programme qui est conçu pour, lorsqu'il est exécuté, réaliser le procédé mis en œuvre par ordinateur selon l'une des revendications 1 à 4.

13. Flux de données comprenant un code de programme qui est conçu pour, lorsqu'il est exécuté, réaliser le procédé mis en œuvre par ordinateur selon l'une des revendications 1 à 4.

14. Serveur web conçu pour fournir le procédé mis en œuvre par ordinateur selon l'une des revendications 1 à 4 en tant que service web.
